# EUROPÄISCHE PATENTSCHRIFT

(11) **EP 1 934 347 B1**
(45) Veröffentlichungstag und Bekanntmachung des Hinweises auf die Patenterteilung: **10.11.2010**
(21) Anmeldenummer: 06806881.6
(22) Anmeldetag: 29.09.2006
(51) Int. Cl.: C12N 15/10, C07H 1/08

(54) **VERFAHREN UND FORMULIERUNG ZUR EXTRAKTION VON NUKLEINSÄUREN AUS BELIEBIGEN KOMPLEXEN AUSGANGSMATERIALIEN**
METHOD AND FORMULATION FOR THE EXTRACTION OF NUCLEIC ACIDS FROM ANY COMPLEX STARTING MATERIALS
PROCÉDÉ ET FORMULATION PERMETTANT L'EXTRACTION D'ACIDES NUCLÉIQUES À PARTIR DE MATÉRIAUX COMPLEXES QUELCONQUES

(30) Priorität: 29.09.2005 DE 102005047736
(43) Veröffentlichungstag der Anmeldung: 25.06.2008
(73) Patentinhaber: AJ Innuscreen GmbH, 13125 Berlin (DE)
(72) Erfinder: TIMO, Hillebrand, 15366 Hoenow (DE)
(74) Vertreter: Wehlan, Helmut
(86) Internationale Anmeldenummer: PCT/EP2006/066883
(87) Internationale Veröffentlichungsnummer: WO 2007/036564

(56) Entgegenhaltungen:
- EP-A1- 0 512 767
- WO-A-00/34463
- WO-A-01/10554
- WO-A2-02/04620
- DE-A1- 10 253 351
- WILLIS E H ET AL: "Prep-A-Gene: a superior matrix for the purification of DNA and DNA fragments." BIOTECHNIQUES. JUL 1990, Bd. 9, Nr. 1, Juli 1990 (1990-07), Seiten 92-99, XP008073102 ISSN: 0736-6205

## Beschreibung

Gegenstand der Erfindung ist ein universelles und stark vereinfachtes Verfahren sowie eine Formulierung zur Isolierung von Nukleinsäuren aus Nukleinsäuren enthaltenden unterschiedlichsten Ausgangsmaterialien, welches sowohl eine sehr hohe Qualität der zu isolierenden Nukleinsäuren garantiert als auch die Isolierung quantitativer Ausbeuten ermöglicht.

Unter klassischen Bedingungen erfolgt die Isolierung von DNA aus Zellen und Geweben dadurch, dass die Nukleinsäuren enthaltenden Ausgangsmaterialien unter stark denaturierenden und reduzierenden Bedingungen - teilweise auch unter Verwendung von proteinabbauenden Enzymen - aufgeschlossen, die austretenden Nukleinsäurefraktionen über Phenol-/Chloroform-Extraktionsschritte gereinigt und die Nukleinsäuren mittels Dialyse oder Ethanolpräzipitation aus der wässrigen Phase gewonnen werden (Sambrook, J., Fritsch, E.F. und Maniatis, T., 1989, CSH, "Molecular Cloning" ).

Diese "klassischen Verfahren" zur Isolierung von Nukleinsäuren aus Zellen und besonders aus Geweben sind sehr zeitaufwendig (teilweise länger als 48 h ), erfordern einen erheblichen apparativen Aufwand und sind darüber hinaus auch nicht unter Feldbedingungen realisierbar. Außerdem sind solche Methoden auf Grund der verwendeten Chemikalien wie Phenol und Chloroform in einem nicht geringen Maße gesundheitsgefährdend.

Verschiedene alternative Verfahren zur Isolierung von Nukleinsäuren aus unterschiedlichen biologischen Ausgangsmaterialien ermöglichen, die aufwendige und gesundheitsschädigende Phenol-/Chloroform-Extraktion von Nukleinsäuren zu umgehen sowie eine Reduzierung der zeitlichen Aufwendungen zu erreichen.

Alle diese Verfahren basieren auf einer von Vogelstein und Gillespie (Proc. Natl. Acad. Sci. USA, 1979, 76, 615-619) entwickelten und erstmals beschriebenen Methode zur präparativen und analytischen Reinigung von DNA-Fragmenten aus Agarosegelen. Die Methode kombiniert die Auflösung der die zu isolierende DNA-Bande enthaltende Agarose in einer gesättigten Lösung eines chaotropen Salzes (NaJ) mit einer Bindung der DNA an Glaspartikel. Die an die Glaspartikel fixierte DNA wird anschließend mit einer Waschlösung (20 mM Tris HCl [pH 7,2]; 200mM NaCl; 2 mM EDTA; 50% v/v Ethanol) gewaschen und danach von den Trägerpartikeln abgelöst.

Diese Methode erfuhr bis heute eine Reihe von Modifikationen und wird zum gegenwärtigen Zeitpunkt für unterschiedliche Verfahren der Extraktion und Reinigung von Nukleinsäuren aus unterschiedlichen Herkünften angewendet (Marko, M.A., Chipperfield, R. und Birnboim, H.G., 1982, Anal. Biochem., 121, 382-387).

Darüber hinaus existieren heute weltweit auch eine Vielzahl von Reagenziensystemen, vor allem zur Reinigung von DNA-Fragmenten aus Agarosegelen und für die Isolierung von Plasmid DNA aus bakteriellen Lysaten, aber auch für die Isolierung von längerkettigen Nukleinsäuren (genomische DNA, zelluläre Gesamt-RNA) aus Blut, Geweben oder auch Zellkulturen.

Alle diese kommerziell verfügbaren Kits basieren auf dem hinlänglich bekannten Prinzip der Bindung von Nukleinsäuren an mineralische Träger unter Anwesenheit von Lösungen unterschiedlicher chaotroper Salze und verwenden als Trägermaterialien Suspensionen feingemahlener Glaspulver (z.B. Glasmilk , BIO 101, La Jolla, CA), Diatomenerden (Fa. Sigma) oder auch Silicagele. (Diagen, DE 41 39 664 A1).

Ein für eine Vielzahl unterschiedlicher Anwendungen praktikables Verfahren zur Isolierung von Nukleinsäuren ist in US 5,234,809 (Boom) dargestellt. Dort ist ein Verfahren zur Isolierung von Nukleinsäuren aus nukleinsäurehaltigen Ausgangsmaterialien durch die Inkubation des Ausgangsmaterials mit einem chaotropen Puffer und einer DNA-bindenden festen Phase beschrieben. Die chaotropen Puffer realisieren sowohl die Lyse des Ausgangsmaterials als auch die Bindung der Nukleinsäuren an die feste Phase. Das Verfahren ist gut geeignet, um Nukleinsäuren aus kleinen Probenmengen zu isolieren und fmdet speziell im Bereich der Isolierung viraler Nukleinsäuren seine praktische Anwendung.

Spezifische Modifikationen dieser Verfahren betreffen den Einsatz von neuartigen Trägermaterialien, welche für bestimmte Fragestellungen applikative Vorteile zeigen (WO-A 95/34569).

In neueren Patentschriften wird offenbart, dass für die Adsorption von Nukleinsäuren an die dem Fachmann bekannten und eingesetzten silikatischen Materialien auch sogenannte antichaotrope Salze als Bestandteil von Lyse/Bindungspuffer-Systemen sehr effizient und erfolgreich eingesetzt werden können (EP 1 135 479 A). Vorteil dieses Verfahrens ist, dass durch die Umgehung der Verwendung von chaotropen Salzen eine deutlich geringere gesundheitliche Gefährdung von den Extraktionssystemen ausgeht. Allerdings werden für eine effiziente Isolierung von Nukleinsäuren aus einer komplexen biologischen Probe, insbesondere in Hinblick auf eine möglichst ausbeutenstarke Nukleinsäure-Gewinnung, im Lysepuffer wiederum hohe Salzkonzentrationen (> 1.5 M) benötigt. So offenbart die Druckschrift, dass die Verwendung fmdenden Lysepuffer, Salzkonzentrationen zwischen 1,5 M - 3 M enthalten.

In der Offenlegungsschrift DE 43 21 904 A wird ein Verfahren beschrieben, dass bei Kombination von chaotropen Hochsalzpuffern mit alkoholischen Komponenten eine effiziente Isolierung von Nukleinsäuren möglich ist. Die in DE 43 21 904 A ausgewiesenen Lysepuffer enthalten dabei immer Salzkonzentrationen von 4 M - 8 M, insbesondere werden als Salze Guanidinhydrochlorid, Guanidinthiocyanat oder Kaliumjodid eingesetzt. Es ist bekannt, dass diese Salze eine Lyse des Ausgangsmaterials sowie eine potente Inaktivierung nukleolytischer Enzyme realisieren. Nach Lyse des Ausgangsmaterials erfolgt dann die Zugabe eines Alkohols. Die Patentschrift offenbart, dass die Zugabe der alkoholischen Komponenten zum Hochsalz-Lysepuffer eine besonders gute Effizienz der Anbindung der Nukleinsäuren an die eingesetzten silikatischen Filtermaterialien vermittelt. Nachteil der Verwendung von Lysepuffern mit hohen Ionenstärken chaotroper Salze sind aber immer der nur limitierte und auch ineffiziente Einsatz von zusätzlichen proteolytischen Enzymen für einen effiziente Aufschluss komplexer biologischer Proben, die diese Enzyme selbst durch die proteindenaturierenden Wirkung der chaotropen Puffer geschädigt werden. Darüber hinaus sind nachfolgend extensive Waschschritte zur Entfernung der hohen Salzkonzentrationen vom eingesetzten Adsorptionsmaterial notwendig. Dem Fachmann ist bekannt, das chaotrope Salze eine stark inhibitorische Wirkung auf eine Vielzahl von downstream-Applikationen ausüben.

Eine weitere Möglichkeit der Isolierung von Nukleinsäuren wird in der Gebrauchsmusterschrift DE 20 20 7793 U offenbart. Das Verfahren beschreibt die Bindung von Nukleinsäuren an Tonmineralien in Gegenwart eines Salzes eines multivalenten Kations. Das Verfahren soll auf einer Ausbildung einer sog. Kationen-Brücke zwischen der zu isolierenden Nukleinsäure und den Tonmineralien basieren. Die Desorption der zu isolierenden Nukleinsäuren erfolgt dabei nicht - wie bei den anderen bekannten Verfahren - mit Wasser bzw. mit einem Niedrigsalzpuffer, sondern mit einem Elutionsmittel, welches mindestens ein komplexbildendes Agenz, das spezifisch für das verwendete multivalente Kation im Bindungspuffer ist. Nachteil der Erfindung ist insbesondere, dass komplexbildende Komponenten (EDTA; EGTA etc.) als Bestandteile von Elutionspuffern oftmals eine Vielzahl von downstream Applikationen stark inhibieren. Obwohl, wie in dem Gebrauchsmusterschrift ausgeführt, das Verfahren ohne die Verwendung von chaotropen Salzen für die Nukleinsäurereinigung möglich sein soll, enthalten eine Reihe von Puffern der Ausführungsbeispiele aber immer chaotrope Salze (wie z.B. Guanidinsalze). Dies betrifft dabei alle Ausführungsbeispiele, die die Isolierung von Nukleinsäuren aus komplexen biologischen Proben beschreiben. Es scheint somit, dass die Isolierung von Nukleinsäuren aus komplexen Proben nicht ohne chaotrope Salze realisiert werden kann.

EP 0 512 767 A1 offenbart ein Verfahren zur Reinigung von DNA aus einer Lösung, das die Bindung der DNA an eine hydrophile Oberfläche unter Verwendung eines wasserlöslichen organischen Lösungsmittels umfasst, wobei das wasserlösliche organische Lösungsmittel aus 1%-100% Ethanol, 1%-100% lsopropanol und 1%-100% Propanol besteht. Dabei wird als hydrophile Oberfläche Celite-Diatomeen, Siliciumdioxidpolymere, Magnesiumsilicat, Siliciumsticksioffverbindungen, Aluminiurnsilicate oder Siliciumdioxid gewählt.

WO 02/04620 A2 beschreibt ein Verfahren zur Isolierung von Nukleinsäuren aus einer Lösung, mit den folgenden Schritten: a) Adsorbieren der in der Lösung enthaltenen Nukleinsäuren in Gegenwart von Alkali- und/oder Erdalkalisalzen an einer SiO₂-haltigen Oberfläche, b) gegebenenfalls Waschen der an der SiO₂-haltigen Oberfläche adsorbierten Nukleinsäuren mit einem alkoholhaltigen Waschpuffer, und c) Eluieren der Nukleinsäuren mit einer wässrigen Lösung und gegebenenfalls Isolieren der Nukleinsäuren, **dadurch gekennzeichnet, dass** in Schritt a) die Adsorption der Nukleinsäuren an die SiO₂-haltige Oberfläche in Gegenwart von 0,1 bis 3 M Alkali und/oder Erdalkalisalzen und 37 bis 70 Vol-% eines aliphatischen Alkohols erfolgt.

DE 102 53 351 A1 beschreibt ein Verfahren zur Isolierung von Nukleinsäuren aus einer Lösung durch Bindung an eine feste Phase, **dadurch gekennzeichnet, dass** man die Nukleinsäure enthaltende Lösung mit Zusätzen so einstellt, dass sie monovalente und multivalente Kationen sowie einen Alkohol und ggf. weitere Zusätze enthält, sie danach mit der festen Phase in Kontakt bringt, den Träger anschließend ggf. wäscht und die Nukleinsäure von der festen Phase löst. Als multivalente Salzkomponente werden Magnesiumchlorid, Calciumchlorid, Zinkchlorid und/oder Manganchlorid verwendet, und als Alkohol Ethanol oder lsopropanol.

WO 01/10554 A2 beschreibt ein Reagenzienkit für die Isolierung von Nukleinsäuren aus einer biologische, nukleinsäurehaltige Kompartimente enthaltenden Probe, **dadurch gekennzeichnet, dass** er a) negativ geladene Partikel aus einem Polymermaterial, b) ein Reagenz I und c) eine Proteinase-Lösung enthält. Bevorzugt enthält das Reagenz I des erfindungsgemäßen Reagenzienkits als Detergens Natriumdodecylsulfat oder Cetylammoniumbromid (CTAB), vorzugsweise jeweils in einer Menge von 1 bis 10%, bezogen auf das Reagenzvolumen. Als aliphatischer Alkohol ist in Reagenz I vorzugsweise Ethanol enthalten, wobei die Konzentration von mindestens 40 Vol. % beträgt Reagenz I enthält ggf. weitere übliche und geeignete Puffer und/oder Hilfssubstanzen. Beispielhaft für Puffersubstanzen wird TRIS/HCI genannt, Hilfssubstanzen können z. B. Komplexbildner wie EDTA sein. Der pH-Wert der Reagenzien wird vorzugsweise auf ungefähr den physiologischen pH-Wert eingestellt. WO 01/10554 A2 lehrt, dass zur Isolierung von Nukleinsäuren aus Proben enthaltend Hefen, ein Reagenz enthaltend ein Detergens und ein Alkohol entsprechend Reagenz I zu verwenden ist.

Zum Stand der Technik zählen weiterhin die Druckschriften DE 198 56 064 A1, DE 102 53 351 A1, DE 699 08 795 T2 und EP 0 796 327 B1. Darin werden Verfahren zur Isolierung von Nukleinsäuren beschrieben, wobei aber keine antichaotropen Bestandteile verwendet werden.

Die Analyse des Standes der Technik verdeutlicht eindrucksvoll, dass eine Vielzahl von Möglichkeiten existiert, Nukleinsäuren an feste Trägermaterialien, insbesondere mineralische Trägermaterialien auf Siliziumbasis, zu binden, nachfolgend zu waschen und die Nukleinsäuren vom Trägermaterial wieder abzulösen. Es wird auch deutlich, dass für die Isolierung von Nukleinsäuren aus komplexen biologischen Proben auch eine Vielzahl von unterschiedlichen Lysepuffersystemen eingesetzt werden können. Die beschriebenen Lysepuffersysteme enthalten dabei immer auch die Salzkomponenten, die für die notwendige Anbindung der zu isolierenden Nukleinsäuren an die jeweiligen bevorzugten Trägermaterialien essentiell sind, wobei die für die Anbindung der Nukleinsäuren selektierten chaotropen Salze auch gleichzeitig die Lyse des Ausgangsmaterials realisieren. Die verwendeten Salzkonzentrationen der Lysepuffer liegen dabei immer im hochsalzigen Bereich. Dies betrifft auch die Gebrauchsmusterschrift DE 20 20 7793 U, obwohl wie dort explizit aufgeführt, die Anbindung von Nukleinsäuren unter Verwendung multivalenter Kationen und der Nutzung von Tonmineralien unter geringen Konzentrationen der multivalenten Salze erfolgen sollte. Die für die Isolierung beschriebenen Konzentrationen der Guanidinumsalze in dieser Gebrauchsmusterschrift sind die dem Fachmann bekannten Hochsalzkonzentrationen.

Der Erfindung lag deshalb die Aufgabe zu Grunde, die Nachteile der im Stand der Technik beschriebenen Lösungen zu beseitigen.

Die Aufgabe wurde durch ein Verfahren und durch eine Formulierung für die Isolierung und Aufreinigung von Nukleinsäuren aus komplexen Proben - gemäß den Merkmalen der Patentansprüche - gelöst, welches universell einsetzbar ist, unabhängig um welches Ausgangsmaterial es sich handelt und welches im Lysepuffer ohne die bisher immer notwendigen hohen Salzkonzentrationen, die bisher für eine Anbindung von Nukleinsäuren an Trägermaterialien notwendig waren, realisiert werden sollte.

Das erfindungsgemäße Verfahren umfasst folgende Schritte:
1. Der Aufschluss der Probe erfolgt mit einem an sich bekanten Puffer ohne chaotrope oder antichaotrope Bestandteile.
2. Nachfolgend wird der Lyseansatz mit einem Gemisch aus einem Alkohol und einem Detergenz, wobei der Anteil der alkoholischen Komponente im Gemisch aus dem Alkohol und dem Detergenz 20 bis 80% beträgt, und anschließend wird dieses Gemisch mit einer nukleinsäurebindenden festen Phase in Kontakt gebracht.
3. Die feste Phase wird danach ggf. mit an sich bekanten Waschpuffer gewaschen.
4. Die gebundene Nukleinsäure wird mit einem Niedrigsalzpuffer oder mit Wasser von der festen Phase abgelöst.

Überraschenderweise zeigte sich, dass die Kombination an sich bekannter Puffers zum proteolytischen Aufschluss biologischer Proben (Gewebe, Vollblut etc.), bestehend z.B. aus SDS, Tris HCl und EDTA mit einer alkoholischen Komponente oder einem Detergenz, es ermöglicht, nach der Lyse des Ausgangsmaterials, eine Probenukleinsäure an ein mineralisches Glasfaser-Filtermaterial zu binden. Die am Filtermaterial gebundene Nukleinsäure kann mit an sich bekannten Waschpuffer gewaschen, nachfolgend kurz getrocknet und nach Zugabe von Wasser bzw. 10mM Tris HCl vom Glasfasermaterial wieder abgelöst werden.

Als nichtionisches Detergenz kann Tween-20; Tween-80; Triton X-100 etc. (ggf. hohe Konzentration mindestens 10%) oder eines Gemisches von Detergenz/Alkohol (auch wieder hohe Konzentration an Detergenz) eingesetzt werden.

Das bedeutet, dass entgegen den bisher beschriebenen Mechanismen und methodischen Lösungsansätzen, eine Adsorption von Nukleinsäuren an bekannte mineralische Trägermaterialien prinzipiell auch ohne Salze erfolgen kann. Damit ist es möglich, den Aufschluss komplexer biologischer Proben mit einem einfachen und vor allem universell einsetzbaren Lysepuffer, ohne die bisher notwendigen Hochsalzkomponenten durchzuführen.

Sollen größere Mengen an Ausgangsmaterialien für die Isolierung von Nukleinsäuren eingesetzt werden, dann wird aber deutlich, dass die Reinheit der zu isolierenden Nukleinsäuren, gemessen als Ratio 260:280, bei der Kombination des beschriebenen Lysepuffers mit einer alkoholischen Bindungspufferkomponente, oftmals nicht ausreichend ist.

Eine deutliche Steigerung der Reinheit der isolierten Nukleinsäure kann aber überraschenderweise dadurch erreicht werden, dass man die alkoholische Komponente mit einem Salz eines multivalenten, vorzugsweise divalenten Kations, versetzt. Die dazu notwendigen Salzkonzentrationen liegen dabei aber nicht im Hochsalzbereich, wie es dem Fachmann nach dem Stand der Technik bekannt und bisher immer beschrieben worden ist. Vorzugsweise wird Magnesiumchlorid verwendet, da MgCl₂ weder chaotrop noch antichaotrpo wirkt.

Das erfindungsgemäße Verfahren, basierend auf der Kombination eines Lysepuffers ohne Salze, die für die Anbindung der Nukleinsäure an ein Trägermaterial notwendig wären, mit einem Bindungspuffer auf der Basis einer alkoholischen Komponente und einer Salzkomponente eines diavalenten Kations ermöglicht die Isolierung von Nukleinsäuren hoher Qualität aus beliebigen Ausgangsmaterialien. Darüber hinaus kann das Verfahren auf Grund der eingesetzten geringen Salzkonzentrationen bisher notwendige Waschschritte reduzieren, was ein deutliche Reduktion der sog. "hands-on-time" bewirkt.

Das Verfahren ist hocheffizient und zeigt, dass auch die Ausbeuten an zu isolierenden Nukleinsäuren im Vergleich zu den bisher weltweit Verwendung findenden Verfahren und Kits mindestens gleichwertig sind. Setzt man dem Bindungspuffer noch eine Detergenzkomponente zu, so bewirkt diese Kombination noch eine weitere Erhöhung der Ausbeuten an den zu isolierenden Nukleinsäuren. Der Zusatz eines Detergenzes im Bindungspuffer bewirkt dabei zusätzlich eine deutliche Reduktion der unspezifischen Adsorption von Hämoglobin an das eingesetzte Glasfasermäterial, wenn Nukleinsäuren z.B. aus Vollblutproben isoliert werden sollen. Dieser Effekt wirkt sich ebenfalls positiv auf den Extraktionsprozess aus, da bekannt ist, das besonders bei Verfahren zur Isolierung von Nukleinsäuren aus Vollblutproben multiple Waschritte notwendig sind, um Hämoglobin vom Trägermaterial zu entfernen.

Da die im erfindungsgemäßen Verfahren eingesetzten Lysepuffer keinerlei Komponenten enthalten, die eine Adsorption von Nukleinsäuren ermöglichen, ist es auch möglich, z.B. notwendige Vorfilterprozesse über die selben Filtermaterialien zu realiseren, die auch final für die Adsorption der Nukleinsäuren eingesetzt werden. Dies stellt eine enorme verfahrenstechnologische Vereinfachung dar. So kann z.B. nach Lyse einer Pflanzenprobe das Lysat über eine Glasfaserfiltermatrix als Bestandteil einer Zentrifugationssäule zentrifugiert werden, um unlysierte Pflanzenmaterialien und inhibitorische Komponenten zu entfernen. Das Filtrat wird nachfolgend mit dem Bindungspuffer versetzt und auf eine weitere Zentrifugationssäule mit Glasfasermatrix überführt. Die Nukleinsäuren binden an die Fasern der Glasfasermatrix, werden mit einem alkoholhaltigen Waschpuffer gewaschen und die gebundenen Nukleinsäuren werden dann final von der Glasfasermatrix nach Zugabe von z.B. Wasser eluiert.

Dies gilt auch für solche komplexen biologischen Proben wie Stuhlproben oder u.U. auch Vollblut. Mit den bisher bekannten Systemen und Verfahren, welche im Lysepuffer auch die für die Adsorption der Nukleinsäuren notwendigen Salzkomponenten enthalten, kann eine solche Vereinfachung nicht realisiert werden.

Das erfindungsgemäße Verfahren zeigt überraschenderweise einen weiteren ganz wesentlichen neuen Effekt.
Es ist bekannt, dass die für die Isolierung und Aufreinigung eingesetzten Trägermaterialien (in Kombination mit den bekannten Hochsalzpuffern und ggf. Alkoholen) Glas, Keramiken, Quarz, Silikagele, Aerosile, Diatomenerden etc. sind. Diese Materialien können porös und nichtporös sein. Sie können als Suspensionen oder auch als Fasern, Gele, Wolle oder Matten Bestandteile von z.B. Zentrifugatioriseinheiten (Zentrifugationsfiltersäulen) etc. sein. Dem Fachmann ist auch bekannt, dass die Bindung von Polyanionen, wie z.B. DNA an negative funktionale Oberflächen erfolgen kann. Dieses Basiswissen stellt den wissenschaftlichen Hintergrund für die Verwendung von negativen oder potentiell negativen festen Phasen für die Anbindung von Nukleinsäuren mit den bekannten Hochsalzpuffern dar.

Überaschenderweise zeigt die im erfmdungsgemäßen Verfahren beschriebene Kombination eines Lysepuffers zum Aufschluss der biologischen Probe mit der nachfolgenden Zugabe eines Bindungspuffers, bestehend aus einem Alkohol, einem Salz eines divalenten Kations und einem Detergenz, dass eine effiziente Anbindung von Nukleinsäuren keinerlei spezifische Anforderungen an die einzusetzenden Trägermaterialien stellt. Neben der Möglichkeit der Verwendung von an sich bekannten Trägermaterialien (insbesondere mit negativen funktionalen Ladungen) konnten physikalisch/chemisch gänzlich unterschiedliche Trägermaterialien für die Isolierung und Aufreinigung von Nukleinsäuren eingesetzt werden. Beispielhaft seien hierbei genannt:

positiv geladene Nylon-Membranen, Polysulfon-Membranen, Polyethersulfon-Membranen PVDF-Membranen, Membranen aus Acrylpolymeren, Ionenaustauscher-Membranen, Polyethylenfritten und selbst einfache Filterpapiere, Glassfasermaterialien (z.B. Papierfilter). Dies ist insofern auch sehr überraschend, da eine Vielzahl der beschriebenen Membranen chemisch inerte, neutrale Oberflächen aufweisen, und eigentlich in der Praxis für Filtrationen eingesetzt werden, mit dem Ziel keinerlei Bindungsaffmitäten gegenüber Biomolekülen zu haben.

Darüber hinaus eigenen sich auch Partikel für die Anbindung der Nukleinsäuren mit dem beschriebenen Verfahren (z.B. funktionalisierte magnetische Eisenoxydpartikel, Silicatpartikel etc.).

Die Anbindung und die finale Desorption der Nukleinsäuren an und von diesen ganz unterschiedlichen Trägermaterialien kann dabei unter den gleichen
Lyse/Bindungspufferbedingungen erfolgen.

Diese Beobachtung lässt auf einen völlig neuartigen Mechanismus schließen, über welchen sich der Prozess der Isolierung und Aufreinigung von Nukleinsäuren realisiert.

Es wird aber deutlich, dass die Möglichkeit des Einsatzes von den verschiedensten Trägermaterialien ganz neue Produktentwicklungen auf dem Gebiet der Isolierung und Aufreinigung von Nukleinsäuren ermöglichen wird. Die sinnvolle Kombination von Lyse/Bindungspuffer mit spezifischen Trägermaterialien bietet nunmehr die Möglichkeit völlig neuer Lösungsansätze für die Isolierung von Nukleinsäuren. Dies ist umso interessanter, da Methoden der molekularen Probenvorbereitung im Kontext der sich rasant entwickelnden molekularen Diagnostik in prinzipiell allen Bereichen unseres Lebens immer wesentlicher werden.

Die Erfindung soll nachfolgend anhand von Ausführungsbeispielen näher erläutert werden.

### Ausführungsbeispiele

### Beispiel 1: Isolierung von genomischer DNA aus Gewebeproben unter Verwendung von unterschiedlichen festen Trägermaterialien

Jeweils ca. 5 mg Gewebematerial (Schweineleber) wurden in 400 µl Lysepuffer ( SDS; Tris HCl, pH 8.0, EDTA ) in einem 1.5 ml Reaktionsgefäß unter Zugabe von 25 µl Proteinase K ( 20mg/ml ) bei 50 °C unter kontinuierlichem Schütteln für 30 min inkubiert. Nach Lyse des Ausgangsmaterials wurde der Lyseansatz für 1 min bei Maximalgeschwindigkeit zentrifugiert um unlysierte Bestandteile abzutrennen. Der Überstand wurde nachfolgend mit 400 µl Bindungspuffer ( 50% Isopropanol, 10 % Tween 20, 0.5 M MgCl₂ ) versetzt und gemischt. Der Ansatz wurde dann auf handelübliche Zentrifugations-Filter mit enthaltenen unterschiedlichen Membranen übertragen und über die Membranen zentrifugiert. Folgende Membranen wurden eingesetzt:
1. Glasfasermmembran (Fa. Filtrak)
2. Tissue Quartz (Fa. Pall)
3. Ionenaustauschermembran (Kationenaustauscher; Fa. Pall)
4. Polysulfonmembran (ungeladen; Fa. Pall)
5. Acrylpolymermembran (ungeladen; Fa. Pall)
6. Filterpapier (13 µm; Fa. Roth)

Das Filtrat wurde danach verworfen und die Zentrifugationssäule einmal mit 800 µl Waschpuffer gewaschen (50 mM NaCl; 10 mM Tris HCl; 1 mM EDTA; 70% v/v Ethanol).
Nach Ethanolentfernung durch einen kurzen Zentrifugationsschritt (12.000 rpm für 2min) erfolgte die Elution der Nukleinsäure durch Zugabe von 200µl eines Elutionspuffers (10 mM Tris-HCl; pH 8,5) durch Zentrifugation für

1 min bei 10.000 rpm.

Nachfolgend erfolgte die spektrophotometrische Vermessung der DNA.

Die Ergebnisse sind in der nachfolgenden Tabelle dargestellt. Es wurden jeweils 3 Extraktionen durchgeführt und die Messwerte nach Messung gemittelt.

| **Membran** | **Ausbeute** | **Ratio 260:280** |
|---|---|---|
| Glasfasermembran (Fa. Filtrak) | 23 µg | 1,92 |
| Tissue Quartz (Fa. Pall) | 22 µg | 1,77 |
| Ionenaustauschermembran (Kationenaustauscher; Fa. Pall) | 14 µg | 1,97 |
| Polysulfonmembran (ungeladen; Fa. Pall) | 17 µg | 2,00 |
| Acrylpolymermembran (ungeladen; Fa. Pall) | 20 µg | 1,87 |
| Filterpapier (13 µm; Fa. Roth) | 11 µg | 1,94 |

Die Ergebnisse demonstrieren, dass die Isolierung von Nukleinsären auch sehr effizient und mit hoher Reinheit mit unterschiedlichen Trägermaterialien realisiert werden kann und nicht nur die bisher eingesetzten klassischen silikatischen Materialien geeignet sind. Die Ergebnisse zeigen auch, dass eine hohe Reinheit der Nukleinsäuren auch bei nur einem Waschschritt erreicht worden ist.

### Beispiel 2:

Ca. 50 mg Lebergewebe (Maus) wurden je Ansatz mit 400 µl Lysepuffer one chaotrope und antichtchaotrope Komponenten, enthaltend 1% SDS; 10 mM EDTA und 50 mM Tris HCl sowie 25 µl Proteinase K versetzt und bei 50°C lysiert.

Nach Lyse erfolgte die Zugabe von 400µl eines Alkohol/Detergenz-Gemisches (50% Isopropanol/40% Tween-20) als Bindungspuffer. Lyseansatz und Bindungspuffer wurden mittels Pipette gründlich gemischt.

Überführen der Proben auf eine Zentrifugationssäule mit Glasfaserfiltermaterial und Zentrifugation bei 10.000 x g für 1 min. Verwerfen des Filtrates.

Nachfolgend zweimaliges Waschen mit einem ethanolhaltigen Waschpuffer (70% Ethanol, Natriumchlorid; Tris HCl).

Trocknung der Säule durch Zentrifugation für 2 min bei 10.000 x g.

Elution der DNA durch Zugabe von 200 µl eines Elutionspuffers (10 mM Tris HCl); Zentrifugation bei 5.000 x g für 1min.

Anschließend wurde die isolierte DNA spektrophotometrisch vermessen.

### Ergebnis

| Probe | **Ratio A260:A280** | **Gesamtausbeute an DNA** |
|---|---|---|
| 1 | 1.87 | 77 µg |
| 2 | 1.84 | 74 µg |
| 3 | 1.86 | 73 µg |
| 4 | 1.88 | 78 µg |
| 5 | 1.83 | 72 µg |

### Schlussfolgerung:

Das Verfahren demonstriert eindeutig, das es möglich ist quantitative Mengen an Nukleinsäuren unter der Verwendung eines dem Fachmann bekannten mineralischen Trägermaterials, Glassfaser, auch ohne jegliche Verwendung von sog. chaotropen oder nichtchaotropen Salzen zu isolieren. Alle dem Fachmann bekannten Extraktionsverfahren, welche Nukleinsäuren an eine mineralische feste Phase binden, benötigten die aufgeführten Salze.

### Definitionen

### Chaotrope Bestandteile:

Substanzen, die regelmäßige - auf der Bildung von Wasserstoffbrückenbindungen beruhende - Struktur von flüssigem Wasser zerstören, indem sie die Bildung der zur Solvatation notwendigen H₂O-Käfigstrukturen verhindern. Beispiele für chaotrope Bestandteile sind Thiocyanate, Iodide oder Perchlorate. Sie bewirken die Denaturierung von Proteinen, die Erhöhung der Löslichkeit unpolarer Substanzen in Wasser sowie Zerstörung der hydrophober Wechselwirkung.

### Antichaotrope Bestandteile:

Substanzen, die regelmäßige - auf der Bildung von Wasserstoffbrückenbindungen beruhende - Struktur von flüssigem Wasser verstärken. Beispiele für anti-chaotrope Bestandteile sind Ammonium-, Natrium- oder Kaliumsalze. Sie bewirken keine Denaturierung, sondern die Verstärkung hydrophober Kräfte und die Verstärkung hydrophober Wechselwirkungen.

### Alkoholische Komponente:

Alkoholische Komponenten im Sinne der Erfindung sind alle wasserlöslichen Alkohole, wie Methanol, Ethanol, Propanol, Isopropanol, Ethylenglycol, Polyethylenglycol oder Gycerin.

## Patentansprüche

1. Verfahren zur Isolierung von Nukleinsäuren aus Nukleinsäuren enthaltenden Materialien, umfassend folgende Schritte:
• Aufschluss des Ausgangsmaterials mit einem Puffer, der weder chaotrope noch antichaotrope Bestandteile enthält
• Zugabe von einem Gemisch aus einem Alkohol und einem Detergenz, wobei der Anteil der alkoholischen Komponente im Gemisch aus dem Alkohol und dem Detergenz 20 bis 80% und der Anteil des Detergenzes mindestens 10 % beträgt
• Bindung der Nukleinsäuren an eine feste Phase
• Waschen der gebundenen Nukleinsäuren mit Waschpuffer
• Elution der gebundenen Nukleinsäuren mit einem Niedrigsalzpuffer oder mit Wasser.

2. Verfahren nach Anspruch 1, **dadurch gekennzeichnet, dass** der Anteil der alkoholischen Komponente im Gemisch aus dem Alkohol und dem Detergenz 45 bis 55 % beträgt.

3. Verfahren nach Anspruch 1 oder 2, **dadurch gekennzeichnet, dass** es sich um ein nichtionisches Detergenz handelt.

4. Verfahren nach einem der Ansprüche 1 bis 3, **dadurch gekennzeichnet, dass** als Alkohol wasserlösliche Alkohole, wie Methanol, Ethanol, Propanol, Isopropanol, Ethylenglycol, Polyethylenglycol oder Glycerin eingesetzt werden.

5. Verfahren nach einem der Ansprüche 1 bis 4, **dadurch gekennzeichnet, dass** der Puffer zum proteolytischen Aufschluss biologischer Proben SDS, TrisHCl und EDTA umfasst.

6. Verfahren nach einem der Ansprüche 1 bis 5, **dadurch gekennzeichnet, dass** als feste Phase beliebige Trägermaterialien eingesetzt werden.

7. Verfahren nach Anspruch 6, **dadurch gekennzeichnet, dass** als feste Phase sowohl bekannte negative funktionale Oberflächen als auch positiv geladene Nylon-Membranen, Polysulfon-Membranen, Polyethersulfon-Membranen PVDF-Membranen, Membranen aus Acrylpolymeren, Ionenaustauscher-Membranen, Polyethylenfritten oder einfache Filterpapiere, Glassfasermaterialien sowie magnetische Eisenoxydpartikel oder Silicatpartikel eingesetzt werden.

8. Verfahren nach einem der Ansprüche 1 bis 7, **dadurch gekennzeichnet, dass** für die Vorfiltration und die finale Adsorption der Nukleinsäuren dasselbe Filtermaterial verwendet wird.

9. Formulierung zur Isolierung von Nukleinsäuren aus Nukleinsäuren enthaltenden Materialien, umfassend
• mindestens einen Puffer zum proteolytischen Aufschluss biologischer Proben, der keine chaotropen oder antichaotropen Bestandteile enthält
• mindestens ein Gemisch aus einem Alkohol und einem Detergenz, wobei der Anteil der alkoholischen Komponente im Gemisch aus dem Alkohol und dem Detergenz 20 bis 80% und der Anteil des Detergenzes mindestens 10 % beträgt
• mindestens eine feste Phase
• Wasch- und Elutionspuffer.

10. Formulierung nach Anspruch 9, **dadurch gekennzeichnet, dass** der Anteil der alkoholischen Komponente im Gemisch aus dem Alkohol und dem Detergenz 45 bis 55 % beträgt.

11. Formulierung nach Anspruch 9 oder 10, **dadurch gekennzeichnet, dass** es sich um ein nichtionisches Detergenz handelt.

12. Formulierung nach einem der Ansprüche 9 bis 11, **dadurch gekennzeichnet, dass** die alkoholische Komponente zusätzlich mindestens ein Salz mit einem multivalenten Kation umfasst.

13. Formulierung nach Anspruch 12, **dadurch gekennzeichnet, dass** ein sich bei dem Salz mit einem multivalenten Kation um ein Salz mit einem zweifach positiv geladenes Kation handelt.

14. Formulierungen nach Anspruch 12 oder 13, **dadurch gekennzeichnet, dass** die Konzentration des multivalenten Kations 1,5 mol/l nicht übersteigt.

## Claims

1. A method for isolating nucleic acids from materials containing nucleic acids, and comprising the following steps :
• solubilising the starting material by means of a buffer containing neither chaotropic nor antichaotropic components
• adding a mixture of an alcohol and a detergent with the proportion of the alcoholic component in the mixture of alcohol and detergent being 20 % to 80 %, and the proportion of detergent being at least 10 %.
• linking the nucleic acids to a solid phase
• washing of the linked nucleic acids by means of detergent buffers
• eluting of the linked nucleic acids by means of a low salt buffer or by means of water.

2. A method according to claim 1, **characterised in that** the proportion of the alcoholic component in the mixture from alcohol and detergent is 45 % to 55 %.

3. A method according to claim 1 or 2, **characterised in that** it is a non-ionic detergent.

4. A method according to claim 1 to 3, **characterised in that** water-soluble alcohol such as methanol, ethanol, propanol, isopropanol, ethylene glycol, polyethylene glycol or glycerin are used as an alcohol.

5. A method according to claim 1 to 4, **characterised in that** the buffer for proteolytic solubilisation of biological samples comprises SDS, TrisHCl and EDTA.

6. A method according to claim 1 to 5, **characterised in that** any support materials are used as a solid phase.

7. A method according to claim 6, **characterised in that** not only known negative functional surfaces but also positively charged nylon membranes, polysulfone membranes, polyethersulfone membranes, PVDF membranes, membranes from acrylic polymers, ion exchange membranes, polyethylene frit membranes or simple filter papers, fibre glass materials as well as magnetic ferrous iron oxide particles or silicate particles are used as a solid phase.

8. A method according to claim 1 to 7, **characterised in that** for pre-filtration and for final adsorption of the nucleic acids the same filter material is used.

9. A formulation for isolating nucleic acid from materials containing nucleic acids, comprising
• at least a buffer for proteolytic solubilisation of biological samples which does not contain any chaotropic or antichaotropic components
• at least a mixture out of an alcohol and a detergent with the proportion of the alcoholic component in the mixture of alcohol and detergent being 20 % to 80 %, and the proportion of the detergent being at least 10 %
• at least one solid phase
• detergent and elution buffers.

10. A formulation according to claim 9, **characterised in that** the portion of the alcoholic component in the mixture of alcohol and detergent is 45 % to 55 %.

11. A formulation according to claim 9 or 10, **characterised in that** it is a non-ionic detergent.

12. A formulation according to any one of claims 9 to 11, **characterised in that** the alcoholic component in addition comprises at least one salt with a multivalent cation.

13. A formulation according to claim 12, **characterised in that** the salt with a multivalent cation is a salt with a cation having a double positive charge.

14. A formulation according to claim 12 or 13, **characterised in that** the concentration of the multivalent cation does not exceed 1.5 mol/l.

## Revendications

1. Procédé d'isolation des acides nucléiques à partir des matériaux contenant des acides nucléiques et comprenant les étapes suivantes :
• la dissolution du matériau de base au moyen d'un tampon contenant ni des composants chaotropes ni des composants antichaotropes
• Adjonction d'un mélange d'un alcool et d'un détergent, la part du composant alcoolique dans le mélange de l'alcool et du détergent étant 20 % à 80 % et la part du détergent étant au moins 10 %
• Liaison des acides nucléiques à une phase solide
• Lavage des acides nucléiques liés par des tampons détergents
• Elution des acides nucléiques avec un tampon à sel faible ou avec de l'eau.

2. Procédé selon la revendication 1, **caractérisé en ce que** la part du composant alcoolique dans le mélange de l'alcool et du détergent est de 45 % à 55 %.

3. Procédé selon les revendications 1 ou 2, **caractérisé en ce qu'**il s'agit d'un détergent non ionique.

4. Procédé selon les revendications 1 à 3, **caractérisé en ce que** des alcools solubles dans l'eau comme du méthanol, d'éthanol, du propanol, d'isopropanol, d'éthylène glycol, du polyéthylène glycol ou du glycérine sont utilisés.

5. Procédé selon la revendication l'une quelconque des revendications 1 à 4, **caractérisé en ce que** le tampon pour la dissolution protéolytique des échantillons biologiques comprend SDS, TrisHCl et EDTA.

6. Procédé selon les revendications 1 à 5, **caractérisé en ce que** des matériaux porteurs quelconques sont utilisés en tant que phase solide.

7. Procédé selon la revendication 6, **caractérisé en ce que** non seulement des surfaces connues fonctionnelles négatives mais aussi des membranes de nylon chargées positivement, des membranes de polysulfone, des membranes de polyéther sulfones, des membranes PVDF, des membranes en polymère acrylique, des membranes d'échangeur d'ions, des frittes en polyéthylène ou des simples papiers filtres, des matériaux en fibre de verre ainsi que des particules magnétiques en oxyde de fer ou des particules en silicate sont utilisés en tant que phase solide.

8. Procédé selon l'une quelconque des revendications 1 à 7, **caractérisé en ce que** pour la préfiltration et l'adsorption finale des acides nucléiques le même matériel de filtre est utilisé.

9. Formule pour isolation des acides nucléiques à partir des matériaux contenant des acides nucléiques et comprenant :
• au moins un tampon pour la dissolution protéolytique des échantillons biologiques ne contenant pas des composants chaotropes ou antichaotropes
• au moins un mélange d'un alcool et d'un détergent, la part du composant alcoolique dans le mélange de l'alcool et du détergent étant 20 % à 80 % et la part du détergent étant au moins 10 %
• au moins une phase solide
• des tampons détergents et d'élution.

10. Formule selon la revendication 9, **caractérisé en ce que** la part du composant alcoolique dans le mélange de l'alcool et du détergent est de 45 % à 55 %.

11. Formule selon la revendication 9 ou 10, **caractérisé en ce qu'**il s'agit d'un détergent non ionique.

12. Formule selon l'une quelconque des revendications 9 à 11, **caractérisé en ce que** le composant alcoolique comprend en outre au moins un sel avec un cation polyvalent.

13. Formule selon la revendication 12, **caractérisé en ce que** le sel avec un cation polyvalent est un sel avec un cation portant une double charge positive.

14. Formule selon les revendications 12 ou 13, **caractérisé en ce que** la concentration du cation polyvalent ne dépasse pas 1,5 mol/l.
